# EUROPEAN PATENT SPECIFICATION

(11) **EP 1 226 827 B1**
(45) Date of publication and mention of the grant of the patent: **02.05.2007**
(21) Application number: 01830043.4
(22) Date of filing: 25.01.2001
(51) Int. Cl.: A61K 36/48, A61K 31/7048, A61P 7/10

(54) **PHARMACEUTICAL COMPOSITION COMPRISING RUTIN AND LESPEDEZA CAPITATA EXTRACT FOR TREATING OEDEMA**
PHARMAZEUTISCHE ZUSAMMENSETZUNGEN RUTIN UND LESPEDEZA CAPITATA EXTRAKT ENTHALTEND ZUR BEHANDLUNG VON ÖDEMEN
COMPOSITION PHARMACEUTIQUE CONTENANT DE LA RUTINE ET UN EXTRAIT DE LA LEPEDEZA CAPITATA POUR LE TRAITEMENT DES OEDEMES

(43) Date of publication of application: 31.07.2002
(73) Proprietor: ROEDER 1956 FARMACEUTICI S.p.A., 10126 Torino (IT)
(72) Inventor: Villa, Claudio, 10131 Torino (IT)
(74) Representative: Gerbino, Angelo

(56) References cited:
- EP-A- 0 699 443
- WO-A-97/28814
- FR-A- 2 770 132
- US-A- 4 460 578
- LINARD A. ET AL: "[Flavonoids from Lespedeza capitata Michx.]. SUR LES FLAVONOIDES DU LESPEDEZA CAPITATA MICHAUX." PLANTES MEDICINALES ET PHYTOTHERAPIE, (1978) 12/2 (144-147). CODEN: PLMPA, XP001007604
- WAGNER, H. ET AL: "Flavonoids in Lespedeza capitata" PHYTOCHEMISTRY (1972), 11(4), 1518 , XP001007949
- "Rote Liste" 1994 , BUNDESVERBAND DER PHARMAZEUTISCHEN INDUSTRIE , FRANKFURT / MAIN XP002172057 * example 81168 *
- STAMPOULIS P ET AL: "Staminol A, a Novel Diterpene from Orthosiphon stamineus" TETRAHEDRON LETTERS,NL,ELSEVIER SCIENCE PUBLISHERS, AMSTERDAM, vol. 40, no. 22, 28 May 1999 (1999-05-28), pages 4239-4242, XP004164707 ISSN: 0040-4039

## Description

The present invention relates in general terms to the therapeutic application of active agents derived from plants.

LINARD A. ET AL: "[Flavonoids from Lespedeza capitata Michx.]. SUR LES FLAVONOIDES DU LESPEDEZA CAPITATA MICHAUX". PLANTES MEDICINALES ET PHYTOTHERAPIE, (1978) 12/2 (144-147). CODEN: PLMPA, XP001007604 discloses an alcoholic extract derived from Lespedeza capitata as being useful as an hypoazotemic agent.

WAGNER, H. ET AL.: "Flavonoids in Lespedeza capitata" PHYTOCHEMISTRY (1972), 11(4), 1518, XP001007949 refers to the plant Lespedeza capitata, comprising i.a. Lespecapitoside and quercetin-3-rhamnoglucoside (rutin).

US-A-4 460 578 refers to flavonoid extracts of lespedeza capitata demonstrating hypoazotemic and hypocholesterinemic properties.

"Rote Liste" 1994, BUNDESVERBAND DER PHARMAZEUTISCHEN INDUS-TRIE, FRANKFURT/MAIN, XP00217205 discloses the drug Lespenephryl^{R} Tropfen, comprising an ethanolic extract of Lespedeza capitata and water (1:5).

WO 97/28814 A discloses cosmetic and pharmaceutical compositions comprising extracts of i.a. the plant Lespedeza capitata.

The present invention relates to an anti-oedema composition, in particular for pharmaceutical use, which includes rutin and/or a water-soluble derivative thereof and an extract of Lespedeza Capitata obtained with an aqueous alcohol solvent.

Lespedeza Capitata is a bush of the Leguminosae-Papilionaceae family. The genus Lespedeza includes 152 species, of which only a few types have significant pharmacological properties. Lespedeza Capitata (Bush Clover) is common in the United States and in Asia and an attempt was once made to establish it in Italy for animal fodder. The parts of the plant used for medicinal purposes are the leaves and young stems which contain significant amounts of flavone glucosides.

In the context of the present invention it proved crucial to use an aqueous-alcohol solvent to obtain the Lespedeza Capitata extract. This solvent can be e.g. an ethanol/water mixture, with the ratio of ethanol to water being between 10:1 and 1:5 and preferably 2:1.

The use of such solvents makes it possible to obtain an extract having a favourable chromatographic profile, with regard both to the total percentage of flavonoids and to the quality of their composition.

The total concentration of flavonoids in the extract should preferably be of at least 1% with a concentration of Lespecapitoside of at least 0.05% and should ideally have a total concentration of at least 12% of flavonoids and a concentration of Lespecapitoside of at least 1%.

Rutin is a bioflavonoid which was first found in Rue or Bitterwort (Ruta Graveolens) but is more commonly extracted from another plant species, Sophora Japonica (Japanese Pagoda tree), which contains it in percentages of around 15-20%. It has already been used medically for its vitamin P content in products having a capillarotropic and venotropic effect. It has been shown to have a clear antioxidant (anti-lipoperoxidant) and anti-inflammatory action on vascular walls.

All water-soluble derivatives of rutin may be used within the scope of the present invention. Many of these, while not working in quite the same way as rutin, are used medically for the same therapeutic purposes.

In particular, derivatives into which polar groups, for example hydroxyl, have been introduced or rutin salts may be used. Preferred examples of such derivatives consist of rutin sulphate, hydroxy-ethylrutoside (troxerutine), 4(G)-alpha-glucopyranosyl-rutin and mixtures thereof.

The composition of the invention has the ability to redress alterations in the body's composition with regard to the values of extracellular mass and extracellular water. Thanks to its anti-oedema properties, it can therefore be used in the treatment of water retention due to various causes, of PMS-related imbalances, of menopausal symptoms and of hydrosaline imbalances in both men and women, since it is able to reduce certain factors responsible for these conditions.

With reference to an individual of an average weight of 60 kg, the composition was found to be effective when a daily dose containing at least 1 mg of rutin and/or a derivative thereof and at least 10 mg of aqueous-alcohol extract of Lespedeza Capitata was given.

Provided at least these minimum doses were given, all ratios between the quantities of the two basic components were found to be equally effective.

The composition of the invention could also include additional plant extracts with diuretic properties, such as Sambucus Nigra (European Elder) and Ortosiphon Stamineus (Java Tea or Cat's Whiskers) in particular, as well as excipients which are pharmaceutically, dietetically and cosmetically acceptable and/or other active ingredients.

According to a variant of the invention, one or both components could be carried in a phospholipidic complex.

The composition of the invention can be administered by any conventional method, orally, topically or transdermally for example, and preferably in doses of around 400 mg/day of Lespedeza Capitata extract and 40 mg/day of rutin and derivatives thereof, taking as reference a person weighing 70 kilograms.

### TOXICOLOGICAL TESTS

As a result of tests aimed at ascertaining any toxic properties of the composition of the invention, carried out by administering it orally to rats, the said composition was found to have an LD50 of over 10g/kg.

### CLINICAL TRIALS ON THE COMBINATION OF LESPEDEZA AND RUTIN

A clinical trial was carried out to determine the effectiveness of the composition of the invention.

Such trial was double blind, with the use of a placebo, on 73 volunteers, of both sexes and aged between 20 and 60 years old. 45.2% of such volunteers were women with normal blood pressure who were not using oral contraceptives, 32.8% were women with normal blood pressure who were using oral contraceptives and 22% were sedentary men with normal blood pressure.

For 30 consecutive days, the volunteers took two tablets a day each having the following composition:
- Lespedeza Capitata (dry extract) containing 12% of total flavonoids and 1% of Lespecapitoside 200 mg
- Rutin 25 mg
- Sambucus Nigra (dry extract) 75 mg
- Ortosiphon Stamineus (dry extract) 75 mg
- Potassium 25 mg
- Vitamin B6 1 mg

The following parameters were measured in the volunteers at the beginning, halfway through and at the end of the trial:
- extracellular mass,
- extracellular water,
- intracellular water,
- Na/K ratio,
- volume of urine excreted in 24 hours,
- electrolyte concentration in urine.

In addition, any cases of oedema which occurred during the trial were kept under observation and evaluated.

An impedentiometric method was used to carry out these measurements, with the assistance of an STA/BIA Akern instrument. This made it possible to measure body fat, lean tissue, basal metabolism and extracellular mass and water without being limited by the use of anthropometric data (weight and height).

The data thus obtained were processed using standard statistical methods (Wilcoxon T test with 95% probability).

The conclusion was that the composition on trial was effective in reducing oedema in the subjects treated, reducing the extracellular mass by 4.5% and extracellular water by 5.06%. Accompanied by an increase in intracellular water of 1.02% and a reduction in the Na/K ratio of 4.3%, these data indicated that the treatment led to a significant improvement, reducing factors which favoured water retention and thus any tendency towards oedema. In addition, measurement of the total volume of urine produced over 24 hours, carried out for a smaller group of subjects (20) characterised by a higher value of extracellular water, showed an increase of 7.9%, suggesting that the composition also had a diuretic effect.

Naturally, the principle of the invention remaining unchanged, embodiments and manufacturing details may vary widely from those described purely by way of non-limitative example, without departing thereby from the scope of the invention. In particular, such embodiments could include the application of the composition of the invention to other fields: in dietetics, food and cosmetics for example.

## Claims

1. Use of a composition which includes rutin and/or a water-soluble derivative thereof selected from the group consisting of rutin sulphate, hydroxy-ethylrutoside (troxerutine), 4(G)-alpha-glucopiranosil-rutin and mixtures thereof, and an extract of Lespedeza Capitata obtainable using an aqueous-alcohol solvent for the manufacture of a medicament for anti-oedema application.

2. A use according to Claim 1, **characterised in that** the said Lespedeza Capitata extract contains at least 1% of total flavonoids and at least 0.05% of Lespecapitoside.

3. A use according to Claim 2, **characterised in that** the said Lespedeza Capitata extract contains at least 12% of total flavonoids and at least 1% of Lespecapitoside.

4. A use according to any preceding Claim, **characterised in that** the said extract is obtainable with the use of an ethanol/water mixture as solvent.

5. A use according to Claim 4, **characterised in that** the ethanol to water ratio in this mixture is between 10:1 and 1:5 and is preferably 2:1.

6. A use according to any preceding Claim, **characterised in that** said composition also contains an additional plant extract with diuretic properties, such as Sambucus Nigra and Ortosiphon Stamineus in particular.

7. A use according to any one of the preceding Claims, **characterised in that** at least one component of said composition is carried in a phospholipidic complex.

8. A use according to any one of the preceding Claims, **characterised in that** said composition contains at least 1 mg of rutin and/or a derivative thereof selected from the group consisting of rutin sulphate, hydroxyethylrutoside (troxerutine), 4(G)-alpha-glucopiranosil-rutin and mixtures thereof and at least 10 mg of Lespedeza Capitata extract.

9. A use according to any one of the preceding Claims, **characterised in that** said composition also includes excipients which are pharmaceutically, dietetically and cosmetically acceptable and/or other active ingredients.

10. A use according to any one of the preceding Claims, wherein said composition is in a form for oral, topical or transdermal administration.

## Patentansprüche

1. Verwendung einer Zusammensetzung, die Rutin und/oder ein wasserlösliches Derivat davon, ausgewählt aus der Gruppe bestehend aus Rutinsulfat, Hydroxyethylrutosid (Troxerutin), 4(G)-alpha-Glucopyranosylrutin und Mischungen davon, und einen mittels eines wässrig-alkoholischen Lösungsmittels erhältlichen Extrakt aus Lespedeza capitata enthält, für die Herstellung eines Medikaments zur ödemhemmenden Anwendung.

2. Verwendung nach Anspruch 1, **dadurch gekennzeichnet, dass** der Lespedeza-capitata-Extrakt wenigstens 1% Gesamtflavonoide und wenigstens 0,05% Lespecapitosid enthält.

3. Verwendung nach Anspruch 2, **dadurch gekennzeichnet, dass** der Lespedeza-capitata-Extrakt wenigstens 12% Gesamtflavonoide und wenigstens 1% Lespecapitosid enthält.

4. Verwendung nach einem der vorhergehenden Ansprüche, **dadurch gekennzeichnet, dass** der Extrakt unter Verwendung eines Ethanol/Wasser-Gemisches als Lösungsmittel erhältlich ist.

5. Verwendung nach Anspruch 4, **dadurch gekennzeichnet, dass** das Verhältnis von Ethanol zu Wasser in diesem Gemisch zwischen 10:1 und 1:5 beträgt und vorzugsweise 2:1 ist.

6. Verwendung nach einem der vorhergehenden Ansprüche, **dadurch gekennzeichnet, dass** die Zusammensetzung außerdem einen zusätzlichen Pflanzenextrakt mit harntreibenden Eigenschaften, wie insbesondere Sambucus nigra und Orthosiphon stamineus, enthält.

7. Verwendung nach einem der vorhergehenden Ansprüche, **dadurch gekennzeichnet, dass** wenigstens ein Bestandteil der Zusammensetzung in einem Phospholipid-Komplex getragen wird.

8. Verwendung nach einem der vorhergehenden Ansprüche, **dadurch gekennzeichnet, dass** die Zusammensetzung wenigstens 1 mg an Rutin und/oder einem Derivat davon, ausgewählt aus der Gruppe bestehend aus Rutinsulfat, Hydroxyethylrutosid (Troxerutin), 4(G)-alpha-Glucopyranosylrutin und Mischungen davon, und wenigsten 10 mg Lespedeza-capitata-Extrakt enthält.

9. Verwendung nach einem der vorhergehenden Ansprüche, **dadurch gekennzeichnet, dass** die Zusammensetzung außerdem Exzipientien, die pharmazeutisch, diätetisch und kosmetisch annehmbar sind, und/oder weitere Wirkstoffe einschließt.

10. Verwendung nach einem der vorhergehenden Ansprüche, wobei die Zusammensetzung in einer Form zur oralen, topischen oder transdermalen Verabreichung vorliegt.

## Revendications

1. Utilisation d'une composition qui comprend de la rutine et/ou son dérivé hydrosoluble choisi dans le groupe consistant en sulfate de rutine, hydroxyéthylrutoside (troxérutine), 4(G)-alpha-glucopiranosil-rutine et leurs mélanges, et un extrait de Lespedeza Capitata pouvant être obtenu en utilisant un solvant alcoolique aqueux pour la fabrication d'un médicament destiné à une application anti-oedème.

2. Utilisation selon la revendication 1, **caractérisée en ce que** ledit extrait de Lespedeza Capitata contient au moins 1 % de flavonoïdes totaux et au moins 0,05 % de Lespecapitoside.

3. Utilisation selon la revendication 2, **caractérisée en ce que** ledit extrait de Lespedeza Capitata contient au moins 12 % de flavonoïdes totaux et au moins 1 % de Lespecapitoside.

4. Utilisation selon l'une quelconque des revendications précédentes, **caractérisée en ce que** ledit extrait peut être obtenu avec l'utilisation d'un mélange éthanol/eau en tant que solvant.

5. Utilisation selon la revendication 4, **caractérisée en ce que** le rapport entre l'éthanol et l'eau dans ce mélange se situe entre 10:1 et 1:5, et il est de préférence de 2:1.

6. Utilisation selon l'une quelconque des revendications précédentes, **caractérisée en ce que** ladite composition contient également un extrait végétal supplémentaire avec des propriétés diurétiques, tel que Sambucus Nigra et Ortosiphon Stamineus en particulier.

7. Utilisation selon l'une quelconque des revendications précédentes, **caractérisée en ce qu'**au moins un composé de ladite composition est porté dans un complexe phospholipidique.

8. Utilisation selon l'une quelconque des revendications précédentes, **caractérisée en ce que** ladite composition contient au moins 1 mg de rutine et/ou son dérivé choisi dans le groupe consistant en sulfate de rutine, hydroxyéthylrutoside (troxérutine), 4(G)-alpha-glucopiranosil-rutine et leurs mélanges, et au moins 10 mg d'extrait de Lespedeza Capitata.

9. Utilisation selon l'une quelconque des revendications précédentes, **caractérisée en ce que** ladite composition comprend également des excipients qui sont pharmaceutiquement, diététiquement et cosmétiquement acceptables et/ou d'autres ingrédients actifs.

10. Utilisation selon l'une quelconque des revendications précédentes, dans laquelle ladite composition se présente sous une forme pour l'administration orale, topique ou transdermique.
